# EUROPÄISCHE PATENTSCHRIFT

(11) **EP 1 570 800 B1**
(45) Veröffentlichungstag und Bekanntmachung des Hinweises auf die Patenterteilung: **11.04.2007**
(21) Anmeldenummer: 04004652.6
(22) Anmeldetag: 01.03.2004
(51) Int. Cl.: A61B 19/00, G06T 7/60, G06T 7/00, A61B 6/02

(54) **Verfahren und Vorrichtung zum Bestimmen einer Symmetrieebene eines dreidimensionalen Objektes**
Method and device for determining the symmetrical plane of a three dimensional object
Méthode et dispositif pour déterminer le plan de symétrie d'un objet tridimensionnel

(43) Veröffentlichungstag der Anmeldung: 07.09.2005
(73) Patentinhaber: BrainLAB AG, 85551 Kirchheim/Heimstetten (DE)
(72) Erfinder: Lachner, Rainer, 85586 Poing (DE); Tuma, Gregor, 81675 München (DE); Vilsmeier, Stefan, 6330 Kufstein (AT)
(74) Vertreter: Schwabe - Sandmair - Marx

(56) Entgegenhaltungen:
- WO-A-99/59106
- WO-A-02/062248
- WO-A-03/060827
- DE-A- 10 137 655
- US-A- 5 799 055
- US-A1- 2003 153 829
- US-B1- 6 359 960

## Beschreibung

Die Erfindung bezieht sich auf ein Verfahren und eine Vorrichtung zum Bestimmen einer Symmetrieebene eines bevorzugt zumindest zum Teil symmetrischen dreidimensionalen Objektes, wie zum Beispiel der mittleren oder Mittsagittal-Ebene einer Hüfte.

Die Bestimmung der Lage der Mittsagittal-Ebene ist zum Beispiel in der Hüftchirurgie von großer Bedeutung, wenn eine neue Gelenkpfanne eingesetzt werden soll, deren exakte Positionierung unter anderem von der Lage der Mittsagittal-Ebene abhängt.

Bei medizinischen und nicht-medizinischen Verfahren ist es häufig erwünscht, dass die Lage einer Symmetrieebene eines zumindest zum Teil symmetrischen Körpers ermittelt werden kann. Insbesondere ist es bei der kranialen und extrakranialen bildgeführten Chirurgie (image guided surgery; IGS) häufig wichtig die sogenannte Mittsagittal-Ebene zu bestimmen. Die Bestimmung dieser Ebene ist relativ leicht, wenn ein dreidimensionales Bild der betrachteten Struktur verfügbar ist, welches zum Beispiel durch eine Computertumorgrafie- oder Kernspinresonanz-Aufnahme gewonnen werden kann. Verfahren zum Ermitteln der Lage einer Symmetrieebene aus dreidimensionalen Strukturen sind im Stand der Technik bekannt, wobei im Wesentlichen versucht wird herauszufinden welche symmetrischen Strukturen des dreidimensionalen Körpers einander entsprechen, um hieraus die Symmetrieebene zu berechnen.

Weiterhin ist es bekannt, dass zum Bestimmen der Mittsagital-Ebene der Hüfte zwei charakteristische oder sogenannte Spinalpunkte auf der Hüfte festgelegt werden, was jedoch häufig problematisch ist, da zum Beispiel aufgrund einer Seitenlage eines Patienten oder aufgrund von über der Hüfte angeordneten Gewebsschichten kein einfacher Zugang zum Beispiel mittels eines Pointers zu diesen charakteristischen Punkten möglich ist. WO-A-02062248 offenbart eine Vorrichtung und ein Verfahren zur Bestimmung einer Symmetrieebene einer dreidimensionalen Objektes aus mindestens zwei zweidimensionalen Aufnahmen des Objektes.

Es ist eine Aufgabe der vorliegenden Erfindung ein Verfahren und eine Vorrichtung zum Bestimmen der räumlichen Lage einer Symmetrieebene eines bevorzugt zumindest zum Teil symmetrischen dreidimensionalen Objektes vorzuschlagen, welche es ermöglichen, dass eine Symmetrieebene des Objektes einfacher bestimmt werden kann.

Diese Aufgabe wird durch ein Verfahren und eine Vorrichtung wie in den unabhängigen Ansprüchen definiert gelöst. Vorteilhafte Ausführungsformen ergeben sich aus den abhängigen Ansprüchen.

Bei dem erfindungsgemäßen Verfahren zum Bestimmen einer Symmetrieebene eines bevorzugt zumindest zum Teil der vollständig symmetrischen dreidimensionalen Objektes, wie zum Beispiel einem Hüftknochen, einem Schädelknochen oder einer anderen symmetrischen Struktur des Körpers, werden mindestens zwei zweidimensionale Abbildungen oder Bilder des Objektes verwendet, welche zum Beispiel durch Aufnahmen des dreidimensionalen Objektes O aus mindestens zwei verschiedenen Richtungen erhalten werden können.

Es wird mindestens eine charakteristische Kurve oder Linie des dreidimensionalen Projektes in einem der zweidimensionalen Bilder bestimmt, wobei als charakteristische Linie oder Kurve bevorzugt eine symmetrische Kurve des Objektes gewählt wird, welche einfach in dem zweidimensionalen Bild identifiziert werden kann, wie zum Beispiel eine Erhöhung und/oder eine Begrenzung des dreidimensionalen Objektes. Beispielsweise kann bei der Abbildung einer Hüfte der Hüftkamm oder eine Kurve auf einer oberen oder unteren Kante des Schambeines oder Sitzbeines verwendet werden.

Anschließend wird erfindungsgemäß durch eine Verformung oder ein Morphing eine der zwei Abbildungen des dreidimensionalen Objektes so deformiert oder verformt, dass dies so aussieht wie die zweite Abbildung des dreidimensionalen Objektes. Ebenso ist es auch möglich, dass beide Abbildungen des dreidimensionalen Objektes so verformt werden, dass diese einander gleichen. Morphing-Verfahren sind im Stand der Technik bekannt und zum Beispiel in GE Christensen. Deformable shape models for anatomy. Electrical Engineering D.Sc. Dissertation, Washington Universtiy, St. Louis, Missouri, August 1994 beschrieben.

Ist das Morphing durchgeführt worden, so kann die zuvor ermittelte mindestens eine charakteristische Linie in der zweidimensionalen Abbildung des dreidimensionalen Objektes unter Verwendung der beim Morphing gewonnenen Verformungs- oder Deformationsfunktion auf die andere zweidimensionale Abbildung übertragen werden. Somit kann unter Verwendung der Abbildungsdaten mindestens einer charakteristischen Linie des dreidimensionalen Objektes auf mindestens zwei verschiedenen Abbildungen durch Rückprojektion dieser Linie der mindestens zwei zweidimensionalen Abbildungen ermittelt werden, wie der dreidimensionale Verlauf dieser charakteristischen Linien am abgebildeten Objekt O ist. Ist der dreidimensionalen Verlauf einer oder mehrerer charakteristischen Kurven oder Linien bekannt, so kann relativ einfach eine Symmetrieebene zu den so ermittelten dreidimensionalen Kurven gefunden werden, welche dann auch gleichzeitig die Symmetrieebene des abgebildeten dreidimensionalen Objektes ist.

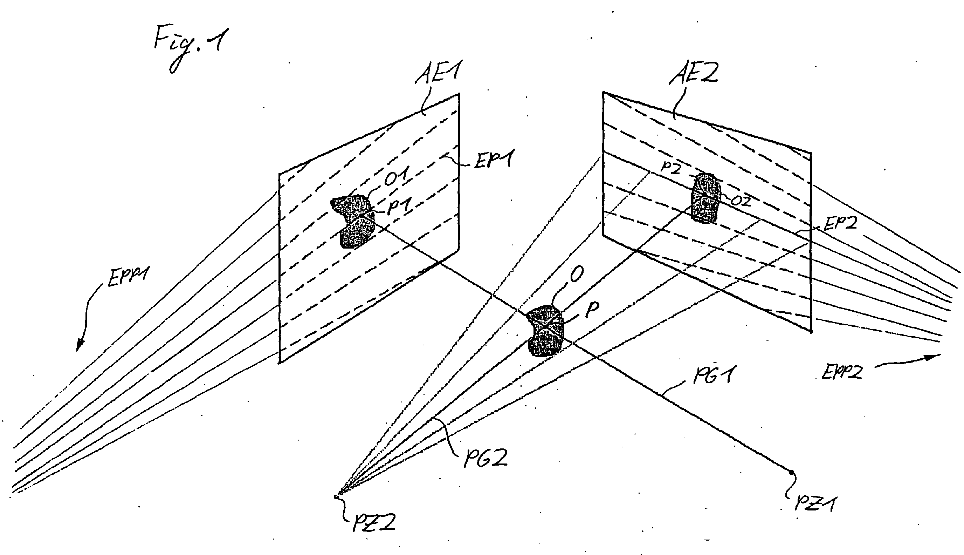

Zum Beispiel wird bei einer Ausführungsform in einem ersten zweidimensionalen Bild des Objekts O mindestens ein Punkt P1 z.B. automatisch oder manuell ausgewählt, welcher aus einer Abbildung eines Objektpunktes P auf eine erste zweidimensionale Abbildungsebene AE1 ausgehend von einem ersten Projektionszentrum PZ1 erzeugt wird, wie schematisch in Figur 1 gezeigt.

Die durch das erste Projektionszentrum PZ1 und den Objektpunkt P definierte erste Projektionsgerade PG1, welche sich im Punkt P1 mit der Bildebene AE1 schneidet, wird durch eine von einem zweiten Projektionszentrum PZ2 durchgeführte Projektion auf eine zweite Bildebene AE2 auf die Epipolar-Gerade EP2 abgebildet, auf welcher der Objektpunkt P2 liegt, welcher die Abbildung des Objektpunktes P auf die zweite Bildebene AE2 ausgehend vom zweiten Projektionszentrum PZ2 ist. Erfindungsgemäß wird bevorzugt unter Verwendung der bekannten räumlichen Anordnung bei der Durchführung der einzelnen Aufnahmen des dreidimensionalen Objektes O, für mehrere oder jeden in der ersten Abbildung liegenden Punkt eine zugeordnete Epipolarlinie in der zweiten Bildebene AE2 erzeugt. Den in der zweiten Abbildung so ermittelten Epipolarlinien werden anschließend korrespondierende Epipolarlinien in der ersten Abbildung zugeordnet, welche als eine Abbildung der von dem zweiten Projektionszentrum PZ2 ausgehenden Projektionsstrahlen, welche Objektpunkte auf der Epipolarlinie der zweiten Abbildung erzeugen können, durch das erste Projektionszentrum PZ1 auf die erste Bildebene AE1 aufgefasst werden können.

Bevorzugt werden die mindestens zwei zweidimensionalen Abbildungen des dreidimensionalen Objektes durch Aufnahmen zum Beispiel mittels Röntgenstrahlen oder sogenannte Fluoroschüsse erzeugt, wobei die Aufnahmen vorteilhaft aus unterschiedlichen Winkeln erfolgen. Vorzugsweise werden die Aufnahmen des Objektes aus zwei verschiedenen Richtungen durchgeführt, wobei die Projektionszentren mit dem Objekt vorzugsweise einen Winkel von 5° bis 30°, also zum Beispiel einen Winkel von 10° einschließen. Es können auch mehrere Aufnahmen des Objektes aus unterschiedlichen Richtungen durchgeführt werden, wobei beispielsweise mit jeweils zwei zweidimensionalen Abbildungen des dreidimensionalen Objektes das oben beschriebene Verfahren durchgeführt wird, um die Genauigkeit bei der Ermittlung der Symmetrieebene des dreidimensionalen Objektes zu erhöhen. Vorteilhaft kann an dem abzubildenden dreidimensionalen Objekt ein Referenzstern oder Marker angebracht sein, um das räumliche Verhältnis des Objektes relativ zu den Projektionszentren und zugeordneten Abbildungsebenen zu ermitteln, wobei diese Lokalisationsdaten zur Durchführung des erfindungsgemäßen Verfahrens verwendet werden können.

Eine erfindungsgemäß verwendete charakteristische Linie oder Kurve des dreidimensionalen Objektes kann zum Beispiel eine Begrenzung oder ein Rand des dreidimensionalen Objektes sein, wie zum Beispiel eine Kurve, welcher auf einer oberen und/oder unteren Kante des Schambeines oder Sitzbeines einer Hüfte liegt. Solche Kurven können automatisch ermittelt werden, wie zum Beispiel durch LiveWire siehe W.A. Barnett, E.N. Motensen. Interactive live-wire boundary extraction. Medical Image Analysis. 1(4):331-341, 1997, LiveWire, siehe M.Kass, A. Witkin, D. Terzopoulos. Snakes: Active contour models. International Journal of Computer Vision, 1(4):321-331, 1988, oder können von einem Benutzer manuell in eine zweidimensionale Abbildung des dreidimensionalen Objektes eingegeben werden. Vorzugsweise werden solche Kurven verwendet, welche eine Symmetrie aufweisen und auf beiden Seiten einer Symmetrieebene einen etwa oder vollständig symmetrischen Verlauf haben.

Nach einer bevorzugten Ausführungsform werden vor der Durchführung des Morphings die mindestens zwei zweidimensionalen Abbildungen rektifiziert, das heißt es werden die in den einzelnen Abbildungen ermittelten Epipolarlinien bevorzugt so parallelisiert, dass eine globale Bildverzerrung minimiert wird. Es werden vorteilhaft die verbleibenden Freiheitsgrade zur Minimierung der Bilderzeugung verwendet.

Die Bestimmung der Symmetrieebene aus dem rekonstruierten dreidimensionalen Verlauf der mindestens einen charakteristischen Kurve oder Linie kann zum Beispiel als nichtlineares least squares Problem angesehen werden, wobei die Symmetrieebene durch bekannte Minimierungsverfahren ermittelt wird, etwa durch das bekannte Levenberg-Marquard-Verfahren. Die Abstandsberechnungen Punkt-Kurve werden durch Verwendung von kd-Bäumen, wie z.B. in J.L.Bentley. Multidimensional Binary Search Trees Used for Associative Searching. Coummun. ACM 18(9):509-517, 1975, stark beschleunigt. -

Weiterhin bezieht sich die Erfindung auf ein Computerprogramm, welches, wenn es in einen Computer geladen wird oder auf einem Computer läuft, einen oder mehrere der oben beschriebenen Verfahrensschritte durchführt, sowie auf eine Programmspeichermedium oder Computerprogrammprodukt mit einem solchen Programm.

Nach einem weiteren Aspekt bezieht sich die Erfindung auf eine Vorrichtung zur Bestimmung der Lage einer Symmetrieebene eines bevorzugt symmetrischen dreidimensionalen Objektes mit mindestens einem Projektionszentrum, wie zum Beispiel einer Strahlenquelle und mindestens einer Projektionsebene, mit welchen eine zweidimensionale Abbildung des dreidimensionalen Objektes erzeugt werden kann. Durch Drehung des Projektionszentrums und/oder der Projektionsebene oder durch Verwendung eines weiteren Projektionszentrums oder einer weiteren Projektionsebene kann eine zur Durchführung des erfindungsgemäßen Verfahrens erforderliche zweidimensionale Abbildung des dreidimensionalen Objektes erzeugt werden, wobei erfindungsgemäß mindestens ein Marker oder Referenzstern vorgesehen ist, welcher an dem Objekt angebracht wird, wodurch das Lageverhältnis des Objektes relativ zu dem mindestens einen Projektionszentrum und/oder der mindestens einen Projektionsebene ermittelt werden kann. Weiterhin ist erfindungsgemäß eine Recheneinheit vorgesehen, welche mindestens zwei zweidimensionale Abbildungsdaten des dreidimensionalen Objektes und die räumlichen Lagedaten des Objekts, des Projektionszentrums und der Abbildungsebene erhält und ein wie oben beschriebenes Verfahren ausführen kann.

Nachfolgend wird die Erfindung anhand eines bevorzugten Ausführungsbeispieles beschrieben.

Es zeigen:
- Figur 1: eine schematische Darstellung der Abbildung eines Objektpunktes auf zwei Abbildungsebenen;
- Figur 2: jeweils zwei zweidimensionale Abbildungen eines dreidimensionalen Objektes, welche aus unterschiedlichen Winkeln aufgenommen wurden;
- Figuren 3 bis 5: jeweils zwei Abbildungen eines Objektes zur Erläuterung der Epipolar-Geometrie;
- Figur 6: eine schematische Darstellung zur Erläuterung des Morphings;
- Figur 7: zwei gemorphte Abbildungen des Objektes mit den in einer Abbildung eingegebenen charakteristischen Linien;
- Figur 8: zwei Abbildungen des Objektes mit den auf die andere Abbildung übertragenen charakteristischen Linien;
- Figur 9: die schematische Darstellung des dreidimensionalen Verlaufes zweier charakteristischer Linien; und
- Figur 10: die Bestimmung der Symmetrieebene zu den in Figur 9 gezeigten charakteristischen Linien.

Figur 1 zeigt schematisch ein Objekt O, welches durch zwei Abbildungen ausgehend von den zwei gezeigten Projektionszentren PZ1 und PZ2 auf zwei Projektions- oder Bildebenen AE1 und AE2 abgebildet bzw. projiziert wird und dort als zweidimensionales abgebildetes Objekt O1 bzw. 02 erscheint. Ein in oder auf dem Objekt O liegender Objektpunkt P wird durch eine von dem Projektionszentrum PZ1 ausgehende Abbildung auf den Bildpunkt P1 abgebildet, wobei die in Figur 1 vom Projektionszentrum PZ1 ausgehende und durch den Objektpunkt P verlaufende Projektionsgerade PG1 beispielsweise ein Röntgenstrahl sein kann, welcher den Bildpunkt P1 auf der Bildebene AE1 erzeugt. Wird der Bildpunkt P1 betrachtet, so kann dieser durch die Abbildung eines Punktes erzeugt worden sein, welcher auf der Projektionsgeraden PG1 liegt, welche durch das Projektionszentrum PZ1 und den Objektpunkt P definiert ist. Die Projektion dieser Projektionsgeraden PG1 ausgehend von dem zweiten Projektionszentrum PZ2 auf die zweite Bildebene AE2 erzeugt auf der Bildebene AE2 die Epipolarlinie EP2, auf welcher der in der Bildebene AE2 liegende dem in der Bildebene AE1 liegenden Bildpunkt P1 entsprechende Bildpunkt P2 liegt. Es können bei Verwendung einer Mehrzahl von in der Bildebene AE1 liegenden Punkten eine Mehrzahl von Epipolarlinien EP2 erzeugt werden, welche in der zweiten Bildebene AE2 liegen. Die einer bestimmten Epipolarlinie EP2 der zweiten Bildebene AE2 zugeordneten Projektionsgeraden PG2 werden durch eine Projektion ausgehend vom ersten Projektionszentrum PZ1 auf korrespondierende Epipolarlinien EP1 auf die Bildebene AE1 abgebildet.

Die Epipolarlinien EP1 schneiden sich in dem ersten Epipol EPP1 und die Epipolarlinien 2 schneiden sich in dem zweiten Epipol EPP2, wobei auf der Verbindungsgeraden der beiden Epipole EPP 1 und EPP 2 die beiden Projektionszentren PZ1 und PZ2 liegen.

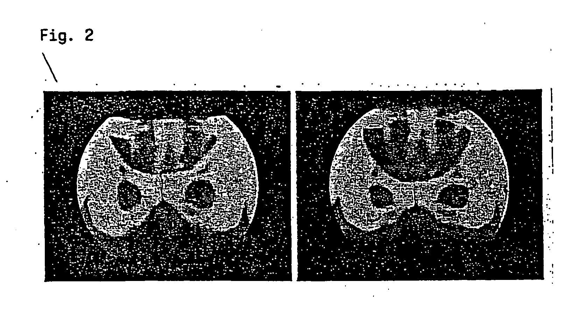

Figur 2 zeigt zwei Röntgenaufnahmen eines Hüftknochens, welche aus unterschiedlichen Richtungen gemacht wurden.

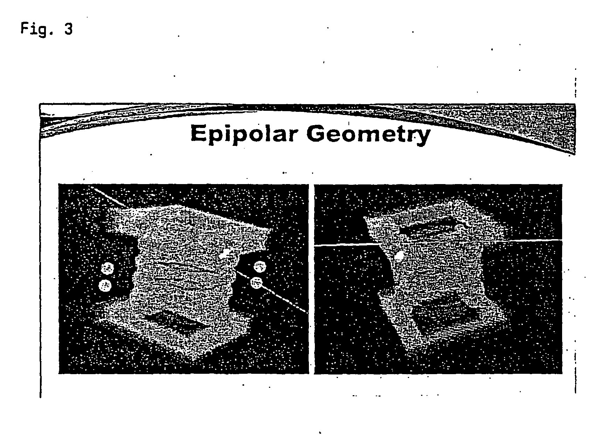

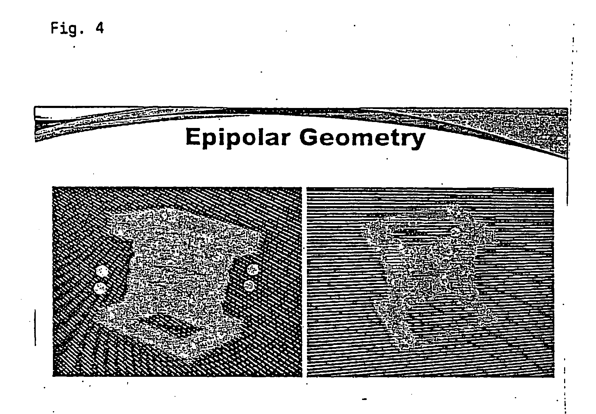

Die erfindungsgemäß verwendete Epipolar-Geometrie wird unter Bezugnahme auf die Figuren 3 bis 5 beschrieben, wobei wie in dem linken Bild von Figur 3 gezeigt, ein Punkt ausgewählt wird und die zu diesem Punkt gehörende Epipolarlinie wie beispielhaft für Figur 1 beschrieben ermittelt wird und im rechten Bild der Figur 3 eingezeichnet ist, auf welcher der zu dem ausgewählten Punkt korrespondierende Punkt liegen muss. Umgekehrt gehört zu einem beliebigen Punkt auf der in dem rechten Bild der in Figur 3 gezeigten Epipolarlinie eine korrespondierende Epipolarlinie im linken Bild. Werden für eine Vielzahl von Punkten die zugehörigen Epipolarlinien in den Bildern erzeugt, können über die gesamten Aufnahmen verteilt Epipolarlinien gefunden werden, wie schematisch in Figur 4 gezeigt, wobei ein Morphing der Bilder nur entlang der Epipolarlinien durchgeführt wird.

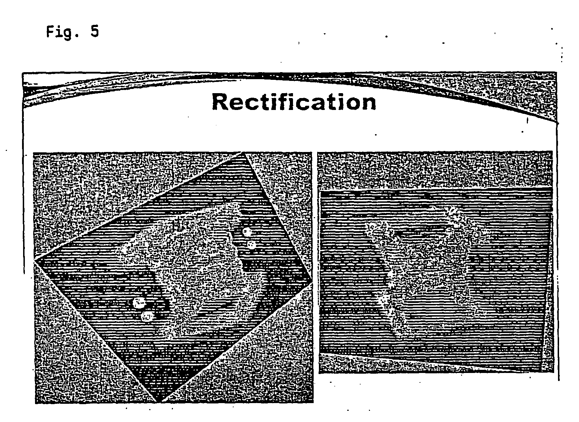

Figur 5 zeigt die in Figur 4 gezeigten Bilder nach dem Durchführen einer Rektifizierung, wobei eine zeilenweise Korrespondenz der Bilder hergestellt wird, so dass ein auf einer Epipolarlinie liegender Punkt einer Abbildung einen korrespondierenden Punkt hat, welcher in der selben Zeile in der anderen Abbildung liegt, wodurch das Morphing effizienter durchgeführt werden kann. Die Rektifizierung kann immer durchgeführt werden, wenn die Epipole außerhalb der Bildsequenzen liegen, was in der Regel der Fall ist, wenn der Winkel, unter welchem die verschiedenen Bilder aufgenommen wurden, nicht zu groß ist.

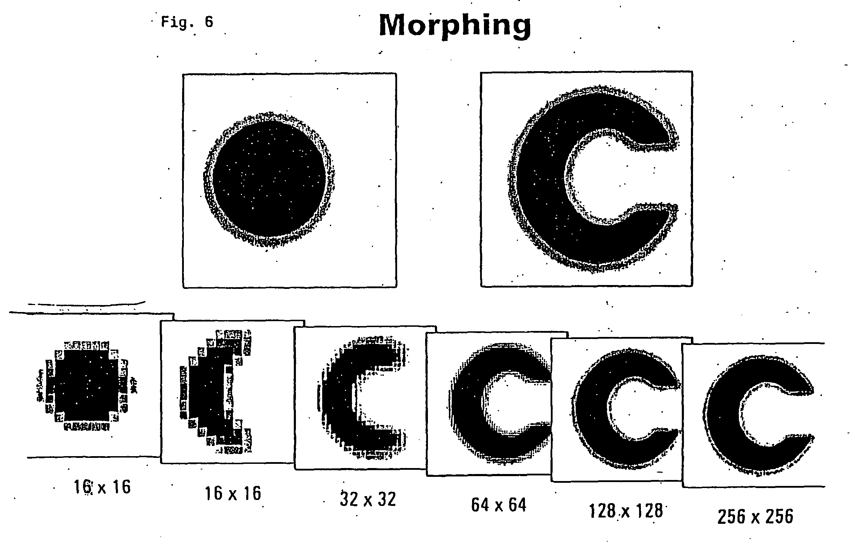

Anschließend wird ein Morphing-Verfahren durchgeführt, also ein elastisches Matching zweier Bilder, wobei ein Bild so deformiert oder verformt wird, dass es zu dem anderen Bild deckungsgleich wird. Figur 6 zeigt beispielhaft das zweidimensionale Matching eines Kreises auf ein großes "C", wobei in einem ersten Schritt die Auflösung auf 16x16 Pixel reduziert wird und das Matching durchgeführt wird. Nach dem erfolgreichen Durchführen des Matchings bei geringer Auflösung wird die Auflösung erhöht und wiederum ein Matching-Verfahren durchgeführt, wobei diese Schritte wiederholt werden, bis die Übereinstimmung der Bilder bei der vollen Auflösung erhalten wird. Es kann durch ein Morphing-Verfahren somit eine Abbildungsvorschrift gefunden werden, durch welche ein Bild auf ein anderes abgebildet wird, wobei die Abbildungsvorschrift auch als Deformationsfeld angesehen werden kann.

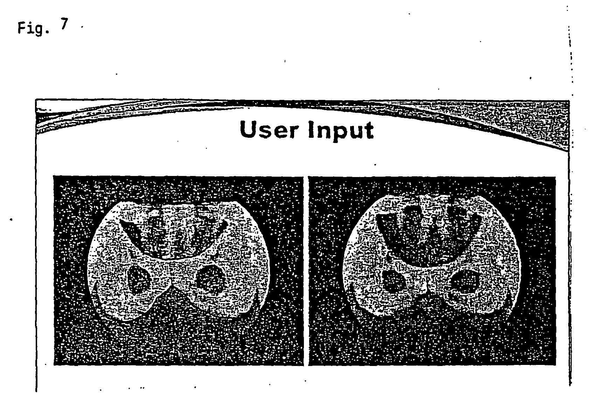

Nach dem Durchführen des Morphings der in Figur 2 gezeigten Abbildungen sind die beiden Aufnahmen der Hüfte etwa deckungsgleich, wie in Figur 7 gezeigt, wobei in einem Bild der Hüfte beispielsweise zwei charakteristische Kurven zum Beispiel manuell oder durch Bildverarbeitungsverfahren eingetragen werden, wie in der linken Abbildung von Figur 7 gezeigt. Diese Kurven verlaufen zum Beispiel entlang der oberen bzw. unteren Kante des Schambeines und Sitzbeines und haben üblicherweise etwa die gleiche Symmetrieebene wie diejenige des dreidimensionalen Objektes.

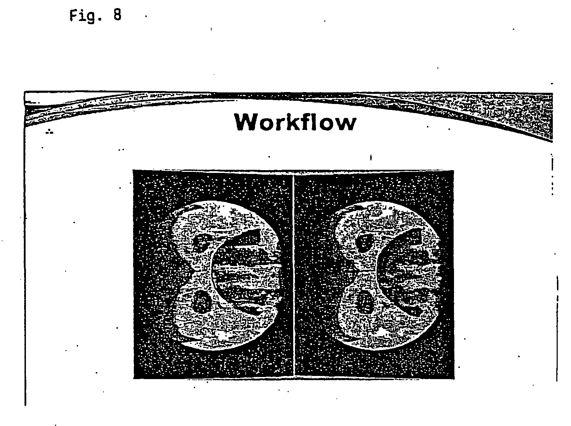

Die bei dem Morphing gefundene Abbildungsvorschrift wird verwendet, um die in dem in Figur 7 im linken Bild eingezeichneten charakteristischen Kurven so zu deformieren, dass diese an den korrespondierenden Punkten der Hüfte im rechten Bild anliegen, wie in Figur 8 gezeigt.

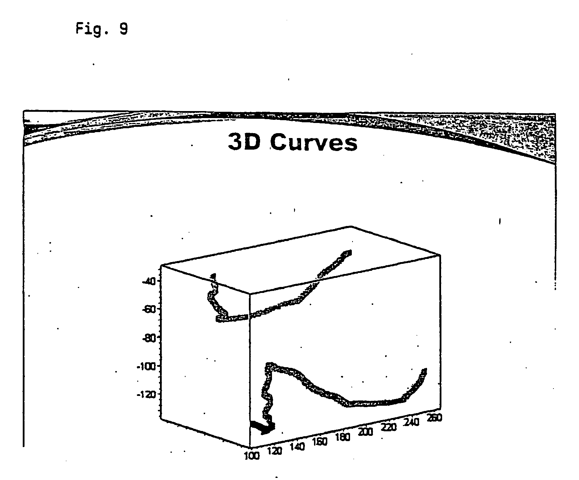

Aus der bei dem Morphing ermittelten Abbildungsvorschrift, dem Verlauf der charakteristischen Kurven in den beiden Abbildungen und den Lokalisationsdaten der Abbildungen können räumliche dreidimensionale Kurven durch Rückprojektion der entsprechenden beiden zweidimensionalen Kurven in den rektifizierten Bildern gewonnen werden, wie schematisch in Figur 9 gezeigt.

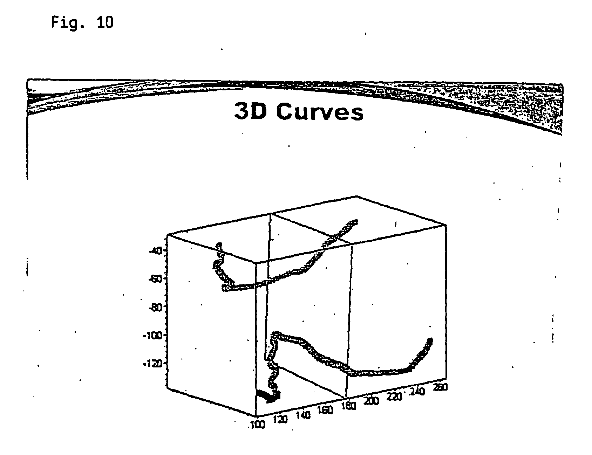

Anschließend wird die Ebene maximaler Symmetrie bezüglich der in Figur 9 gezeigten beiden Kurven ermittelt, wobei die in Figur 10 eingezeichnete Symmetrieebene der Kurven auch gleichzeitig die Symmetrieebene der Hüfte bzw. des aufgenommenen Objektes ist.

## Patentansprüche

1. Verfahren zum Bestimmen der Lage einer Symmetrieebene eines dreidimensionalen Objektes aus mindestens zwei zweidimensionalen Abbildungen des Objektes aus verschiedenen Richtungen, wobei:
a) mindestens eine charakteristische Linie oder Kurve des dreidimensionalen Objektes in einer der zweidimensionalen Abbildungen bestimmt wird;
b) ein Morphing der mindestens zwei zweidimensionalen Abbildungen durchgeführt wird;
c) die mindestens eine charakteristische Linie auf die andere zweidimensionale Abbildung übertragen wird;
d) eine Rückprojektion der charakteristischen Linien aus den zweidimensionalen Abbildungen durchgeführt wird, um eine dreidimensionale den charakteristischen Linien entsprechende Kurve zu erhalten; und
e) eine Symmetrieebene des Objektes aus dem Verlauf der dreidimensionalen Kurve ermittelt wird.

2. Verfahren nach dem vorhergehenden Anspruch, wobei die mindestens zwei zweidimensionalen Abbildungen des Objektes durch Aufnahmen des Objektes aus mindestens zwei verschiedenen Richtungen gewonnen werden.

3. Verfahren nach Anspruch 2, wobei die Abbildungen durch eine oder mehrere Röntgenaufnahmen erzeugt werden.

4. Verfahren nach einem der beiden vorhergehenden Ansprüche, wobei der Aufnahmewinkel im Bereich von 5 bis 20° liegt und insbesondere 10° beträgt.

5. Verfahren nach einem der vorhergehenden Ansprüche, wobei Lokalisationsdaten der Bildaufnahmevorrichtungen verwendet werden.

6. Verfahren nach einem der vorhergehenden Ansprüche, wobei die mindestens eine charakteristische Linie eine Begrenzungslinie und/oder eine vorstehende Linie des dreidimensionalen Objektes ist.

7. Verfahren nach einem der vorhergehenden Ansprüche, wobei eine Rektifizierung vor dem Morphing durchgeführt wird.

8. Computerprogramm, welches, wenn es in einem Computer geladen ist oder auf einem Computer läuft, das Verfahren nach einem der vorhergehenden Ansprüche durchführt.

9. Programmspeichermedium oder Computerprogrammprodukt mit dem Programm nach dem vorhergehenden Anspruch.

10. Vorrichtung zur Bestimmung einer Symmetrieebene eines dreidimensionalen Objektes aus mindestens zwei zweidimensionalen Aufnahmen des Objektes mit mindestens einem Projektionszentrum und mindestens einer dem Projektionszentrum zugeordneten Projektionsebene, mindestens einem Marker, welcher mit dem dreidimensionalen Objekt verbunden werden kann und mit einer Recheneinheit, welcher durch die Marker ermittelte Positionsdaten des Objektes und die Aufnahmedaten des dreidimensionalen Objektes in der mindestens einen Projektionsebene zugeführt werden und welche daraus mit dem Verfahren nach einem der Ansprüche 1 bis 7 die Symmetrieebene des dreidimensionalen Objektes berechnet.

11. Vorrichtung nach dem vorhergehenden Anspruch, wobei das Projektionszentrum eine Strahlenquelle, insbesondere eine Röntgenstrahlenquelle ist.

## Claims

1. A method for determining the position of a plane of symmetry of a three-dimensional object from at least two two-dimensional mappings of said object from different directions; wherein:
a) at least one characteristic line or curve of the three-dimensional object is determined in one of said two-dimensional mappings;
b) the at least two two-dimensional mappings are morphed;
c) said at least one characteristic line is transferred onto the other two-dimensional mapping;
d) the characteristic lines from the two-dimensional mappings are back-projected, in order to obtain a three-dimensional curve corresponding to the characteristic lines; and
e) a plane of symmetry of the object is ascertained from the course of said three-dimensional curve.

2. The method as set forth in the preceding claim, wherein the at least two two-dimensional mappings of the object are obtained using recordings of the object from at least two different directions.

3. The method as set forth in claim 2, wherein the mappings are generated by one or more x-ray recordings.

4. The method as set forth in any one of the preceding two claims, wherein the recording angle is in the range of 5° to 20° and is in particular 10°.

5. The method as set forth in any one of the preceding claims, wherein localisation data of the image recording devices are used.

6. The method as set forth in any one of the preceding claims, wherein the at least one characteristic line is a boundary line and/or a protruding line of the three-dimensional object.

7. The method as set forth in any one of the preceding claims, wherein the images are rectified before they are morphed.

8. A computer program which, when loaded onto a computer or running on a computer, performs the method as set forth in any one of the preceding claims.

9. A program storage medium or computer program product comprising the program as set forth in the preceding claim.

10. A device for determining a plane of symmetry of a three-dimensional object from at least two two-dimensional recordings of said object, comprising: at least one centre of projection and at least one projection plane assigned said centre of projection; at least one marker which can be connected to said three-dimensional object; and a computational unit which is supplied with positional data of the object ascertained by the markers and the recording data of the three-dimensional object in the at least one projection plane, and which calculates the plane of symmetry of the three-dimensional object from these, using the method as set forth in any one of claims 1 to 7.

11. The device as set forth in the preceding claim, wherein said centre of projection is a radiation source, in particular an x-ray radiation source.

## Revendications

1. Procédé pour déterminer la position d'un plan de symétrie d'un objet tridimensionnel à partir d'au moins deux reproductions bidimensionnelles de l'objet depuis différentes directions, dans lequel :
a) au moins une ligne ou courbe caractéristique de l'objet tridimensionnel est déterminée dans l'une des deux reproductions bidimensionnelles ;
b) un morphing des au moins deux reproductions bidimensionnelles est exécuté ;
c) la au moins une ligne caractéristique est reportée sur l'autre reproduction bidimensionnelle ;
d) une rétroprojection des lignes caractéristiques des deux reproductions bidimensionnelles est exécutée afin d'obtenir une courbe tridimensionnelle correspondant aux lignes caractéristiques ; et
e) un plan de symétrie de l'objet est déterminé à partir de l'allure de la courbe tridimensionnelle.

2. Procédé selon la revendication précédente, dans lequel les au moins deux reproductions bidimensionnelles de l'objet sont obtenues par des prises de vue de l'objet depuis au moins deux directions différentes.

3. Procédé selon la revendication 2, dans lequel les reproductions sont produites par une ou plusieurs radiographies.

4. Procédé selon l'une des deux revendications précédentes, dans lequel l'angle de prise de vue est compris entre 5 et 20°, et en particulier égal à 10°.

5. Procédé selon l'une des revendications précédentes, dans lequel on utilise des données de localisation des directions de prise de vue.

6. Procédé selon l'une des revendications précédentes, dans lequel la au moins une ligne caractéristique est une ligne de délimitation et/ou une ligne saillante de l'objet tridimensionnel.

7. Procédé selon l'une des revendications précédentes, dans lequel une rectification est exécutée avant le morphing.

8. Programme d'ordinateur qui met en oeuvre le procédé selon l'une des revendications précédentes lorsqu'il est chargé dans un ordinateur ou se déroule dans un ordinateur.

9. Support d'enregistrement de programme ou produit de programme d'ordinateur contenant le programme selon la revendication précédente.

10. Dispositif pour déterminer un plan de symétrie d'un objet tridimensionnel à partir d'au moins deux prises de vue bidimensionnelles de l'objet avec au moins un centre de projection et au moins un plan de projection associé au centre de projection, au moins un marqueur qui peut être relié à l'objet tridimensionnel et avec une unité de calcul à laquelle sont amenées les données de position de l'objet, déterminées par le marqueur, et les données de prises de vue de l'objet tridimensionnel dans le au moins un plan de projection, et qui calcule, à partir de celles-ci, le plan de symétrie de l'objet tridimensionnel, par le procédé selon l'une des revendications 1 à 7.

11. Dispositif selon la revendication précédente, dans lequel le centre de projection est une source de rayons, en particulier une source de rayons X.
